# EUROPEAN PATENT APPLICATION

(11) **EP 4 473 930 A1**
(43) Date of publication of application: **11.12.2024**
(21) Application number: 23787586.9
(22) Date of filing: 07.04.2023
(51) Int. Cl.: A61B 90/00

(54) **SURGICAL IMPLANTATION IMAGING METHOD AND IMAGING SYSTEM**

(30) Priority: 14.04.2022 CN 202210389241
(71) Applicant: Agloe Medical Technology Co., Ltd., Suzhou, Jiangsu 215413 (CN)
(72) Inventor: YOUNG, Victor, Suzhou, Jiangsu 215413 (CN); GUPTA, Shaurya, Suzhou, Jiangsu 215413 (CN); CHEN, Chaoliang, Suzhou, Jiangsu 215413 (CN); SHI, Weisong, Suzhou, Jiangsu 215413 (CN); SUI, Guangyu, Suzhou, Jiangsu 215413 (CN)
(74) Representative: Keller Schneider Patent- und Markenanwälte AG
(86) International application number: PCT/CN2023/086819
(87) International publication number: WO 2023/197941

(57) **Abstract**

The present application provides an imaging method and imaging system for surgical implantation, and the imaging system includes: a support; a mechanical arm, where a first end of the mechanical arm is rotatably connected to the support; an imaging device, connected to a second end of the mechanical arm, where the imaging device rotates freely under the driving of the mechanical arm, and is used to obtain a light signal reflected by an observation object and perform imaging; a light source providing device, where one end of the light source providing device is provided on the support, and some or all of the light signals provided by the light source providing device are transmitted to the observation object through an surgical incision, and are reflected to the imaging device by the observation object; a retractor, where the retractor is used for placement within the surgical incision to establish a surgical channel, so that the light signal reflected by the observation object can be obtained by the imaging device; and a controller, where the controller is electrically connected to the mechanical arm and the imaging device, makes an adjustment to a posture according to a position and a direction of the retractor, and controls the mechanical arm to drive the imaging device to rotate freely. According to the imaging system of the embodiment of the present application, long-distance shooting can be implemented, and it is conducive to performing the surgery.

## Description

This application claims priority to Chinese Patent Application No. 202210389241.4, filed on April 14, 2022 and entitled "IMAGING METHOD AND IMAGING SYSTEM FOR SURGICAL IMPLANTATION", which is incorporated herein by reference in its entirety.

### Technical field

The present application relates to the field of surgical imaging, and in particular, to an imaging method and an imaging system for surgical implantation.

### Background

Existing electron microscopes for spine surgery require a very short working distance between the surgical field and the microscope objective lens during spine surgery. This results in an uncomfortable viewing angle for the entire surgical team and limited visualization of the surgery. The short working distance and uncomfortable viewing angle also limit the free movement of surgical tools, hindering surgery. As the surgery progresses, the observation axis needs to be adjusted to visualize different parts of the surgical cavity. This process of adjusting the visualization axis is often cumbersome, and most of the time, the surgeon is unable to achieve an optimal setting, exacerbating visualization problems within the surgical cavity. Therefore, there is an urgent need to solve these problems.

### Summary

In view of this, the present application provides an imaging method and an imaging system for surgical implantation, which enables the imaging device to perform imaging away from the working area. This is more conducive to the visualization of the surgical cavity by the surgical team, and it is convenient to perform the surgery.

In order to solve the above technical problems, the present application provides an imaging system for surgical implantation.

According to a first aspect, an embodiment of the present application provides an imaging system for surgical implantation, including:
a support;
a mechanical arm, where a first end of the mechanical arm is rotatably connected to the support;
an imaging device, where the imaging device is connected to a second end of the mechanical arm, the imaging device rotates freely under the driving of the mechanical arm, and is used to obtain a light signal reflected by an observation object and perform imaging, where the light signal reflected by the observation object is transmitted from a surgical incision to the imaging device through a channel established by the retractor, and a lens of the imaging device may be a combination of an optical zoom camera and a digital zoom camera to achieve a greater magnification;
a light source providing device, where one end of the light source providing device is arranged on the support, some or all of the light signals provided by the light source providing device are transmitted to the observation object through the surgical incision, and are reflected by the observation object to the imaging device to assist the imaging device in imaging; and
a controller, where the controller is electrically connected to the mechanical arm, the light source providing device, and the imaging device, and the controller obtains a position and a direction of the surgical incision and makes an adjustment to a posture, and adjusts an aerial posture of the mechanical arm according to a position and a direction of a surgical tool, so that the imaging device can accurately obtain the reflected light signal.

According to the imaging system of the embodiment of the present application, an imaging device is controlled by a mechanical arm, and a light source is provided remotely for the imaging device by using a light source providing device, so that the imaging device can clearly obtain an image of an observation object at a relatively long distance. Compared with the existing microscope, in the present application, the light source is provided closer to the surgical position, and the lighting efficiency is greatly improved, so that the image of the observation object can be clearly obtained when a working distance between the microscope lens and the surgical working area is more than 1 meter, and it is convenient to perform the surgery.

As an embodiment of the first aspect of the present application, the imaging system further includes: a retractor, where the retractor is used for placement within the surgical incision to establish a transmission channel inside and outside the surgical incision; the controller obtains a position and a direction of the retractor and makes an adjustment to a posture, and adjusts an aerial posture of the mechanical arm according to the position and direction of the retractor, so that the imaging device can accurately obtain the reflected light signal from the channel established by the retractor.

As an embodiment of the first aspect of the present application, the imaging device includes: a light filter, where the light filter is used to separate the light signal of a preset wavelength; and an imaging camera, where the imaging camera obtains a wavelength within a preset range, and performs imaging based on the wavelength within the preset range. The light filter in the present application can separate infrared and visible light to achieve imaging in different bands and infrared navigation functions.

As an embodiment of the first aspect of the present application, the mechanical arm has at least six joints to achieve at least six freedom angles of rotation. This structure can implement the free rotation of the mechanical arm in space, avoid the surgical area during adjustment, avoid blocking the line of sight of a surgical operator, and drive the imaging device to better obtain an image of the observation object.

As an embodiment of the first aspect of the present application, the support includes: a fixing frame; and a support frame, where a first end of the support frame is connected to the fixing frame, a second end extends outward toward the side away from the fixing frame, and the second end of the support frame is connected to the mechanical arm, to increase a spatial range that the mechanical arm can reach.

As an embodiment of the first aspect of the present application, the support further includes: a connecting piece, where the connecting piece is fixed on the second end of the support frame, and is used to connect the mechanical arm and the support frame.

As an embodiment of the first aspect of the present application, the light source providing device includes: a light emitting diode LED, where the LED is installed on the fixing frame, a light-emitting surface of the LED and the mechanical arm are located on the same side of the fixing frame, and are used to transmit light to the observation object through the channel established by the retractor.

As an embodiment of the first aspect of the present application, the light source providing device further includes: a light guide, where one end of the light guide is arranged opposite to the LED, and the other end is connected to the retractor, and is used to transmit the light signal emitted by the LED into the retractor, so that the light signal reaches the observation object through the channel. This structure facilitates the introduction of light into the surgical incision and improves the lighting brightness in the surgical incision. In this way, not only it is convenient for the doctor to perform the surgery, but also it is beneficial for the imaging device to clearly obtain the image of the observation obj ect.

As an embodiment of the first aspect of the present application, the imaging system further includes: a display, connected to the controller, and used for displaying the image obtained via the imaging device.

As an embodiment of the first aspect of the present application, there are at least three displays, at least two of the at least three displays are oriented differently, so that the surgical operator can observe the image from different positions, and it is convenient for the surgical team to perform precise operations.

As an embodiment of the first aspect of the present application, the imaging system further includes:
a base, where the support is fixed on the base, and a plurality of rollers are provided below the base.

According to a second aspect, the present application further discloses an imaging method for surgical implantation, where the method is applied to an imaging system, the imaging system includes: a mechanical arm, and an imaging device connected to the mechanical arm, and a controller electrically connected to the mechanical arm and the imaging device, and the method includes: obtaining, by the controller, a position and a direction of a retractor; adjusting, by the controller, a position and a direction of the mechanical arm according to the position and the direction of the retractor, so that an obtaining end of the imaging device is aligned with a channel established by the retractor; where the retractor is used to establish a channel to assist the imaging device in imaging; and obtaining, by the imaging device, a light signal reflected by the observation object through the channel, and performing imaging by using the reflected light signal.

According to the imaging method of the embodiment of the present application, a mechanical arm is controlled to drive an imaging device to obtain an image of an observation object, and a light source providing device remotely provides a light source for the imaging device, so that the imaging device can clearly obtain an image of the observation object at a long distance. Compared with the existing microscope, in the present application, the light source is provided closer to the surgical position, and the lighting efficiency is greatly improved, so that the image of the observation object can still be clearly obtained when a working distance between the microscope lens and the surgical working area is more than 1 meter, and it is convenient to perform the surgery.

As an embodiment of the second aspect of the present application, the imaging method further includes: obtaining, by the imaging device, a light signal of a preset wavelength from the reflected light signal, and performing imaging based on the light signal of the preset wavelength.

As an embodiment of the second aspect of the present application, the imaging system further includes a light source provider, and the light source provider emits a light signal and transmits some or all of the optical signals through the channel to the observation object, so that the imaging device obtains the light signal reflected by the observation obj ect.

As an embodiment of the second aspect of the present application, the imaging system further includes a plurality of displays, and interface orientations of the plurality of displays have at least two or more orientations, and the method further includes: obtaining, by the controller, imaging data obtained by the imaging device, and sending the imaging data to the plurality of displays, so that the plurality of displays with different orientations display the image corresponding to the observation object.

The beneficial effects of the above technical solution of the present application are as follows:
According to the imaging system for surgical implantation according to the embodiment of the present application, by providing a mechanical arm, an imaging device, and a controller, and a connection relationship between various components, according to the imaging system according to the embodiment of the present application, the imaging device is controlled by the mechanical arm, and a light source providing device remotely provides a light source for the imaging device, so that the imaging device can clearly obtain an image of the observation object at a relatively long distance. Compared with the existing microscope, in the present application, the light source is provided closer to the surgical position, and the lighting efficiency is greatly improved, so that the image of the observation object can still be clearly obtained when a working distance between the microscope lens and the surgical working area is more than 1 meter, and it is convenient to perform the surgery.

### Brief description of drawings

FIG. 1 is a schematic three-dimensional structural diagram of an imaging system for surgical implantation according to an embodiment of the present application;
FIG. 2 is a front view of an imaging system for surgical implantation according to an embodiment of the present application;
FIG. 3 is a left view of an imaging system for surgical implantation according to an embodiment of the present application;
FIG. 4 is a right view of an imaging system for surgical implantation according to an embodiment of the present application;
FIG. 5 is a schematic three-dimensional structural diagram of a retractor according to an embodiment of the present application;
FIG. 6 is a front view of a retractor according to an embodiment of the present application;
FIG. 7 is a schematic cross-sectional structural diagram of a retractor according to an embodiment of the present application;
FIG. 8 is a system architecture diagram of an imaging system for surgical implantation according to an embodiment of the present application; and
FIG. 9 is a flow chart of an imaging method for surgical implantation according to an embodiment of the present application.

### Reference signs

Imaging system 100 for surgical implantation;
Support 10; fixing frame 11; connecting piece 2; support frame 13;
Mechanical arm 20; joint 21;
Retractor 30, first channel 31, second channel 32;
Imaging device 40;
Controller 50;
First display 61; second display 62; third display 63;
Light-emitted diode 70;
Base 80; roller 81.

### Detailed description of embodiments

In order to make the objective, technical solution, and advantages of the embodiments of the present application clearer, the technical solution of the embodiments of the present application is clearly and completely described below with reference to the drawings of the embodiment of the present application. Obviously, the described embodiments are some embodiments of the present application, not all of the embodiments. Based on the described embodiments of the present application, all other embodiments obtained by a person having ordinary skill in the art in the art belong to the scope of protection of the present application.

In order to facilitate the understanding of the technical solution of the present application, the technical problem to be solved by the present application is first described.

During surgery, intraoperative imaging, such as C-arm imaging, is often required. Due to the structure and configuration of existing surgical microscopes, the distance between the lens and the surgical area is very short. During surgery, before imaging, the microscope needs to be moved away to perform C-arm imaging; after imaging, the C-arm needs to be moved away, and the microscope needs to be put back. The operation is complicated, and the surgical process is delayed. In addition, because the traditional microscope requires a very short working distance between the surgical area and the microscope objective lens, the intraoperative imaging operation is complicated, the viewing angle of the entire surgical team is uncomfortable, and the visualization of the surgery is limited, affecting the surgical effect.

An imaging system 100 for surgical implantation according to an embodiment of the present application is described in detail with reference to the accompanying drawings.

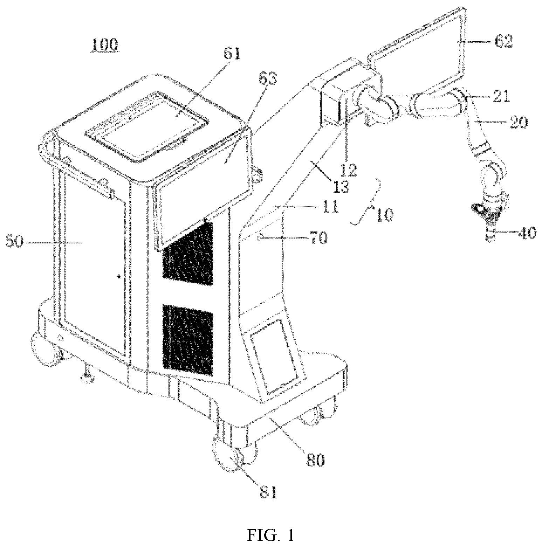

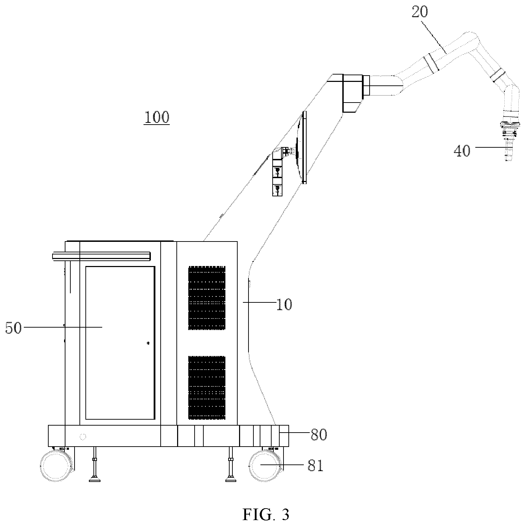

As shown in FIGS. 1-4, the imaging system 100 for surgical implantation according to an embodiment of the present application includes a support 10, a mechanical arm 20, an imaging device 40, a light source providing device, and a controller 50.

Specifically, a first end of the mechanical arm 20 is rotatably connected to the support 10, and a second end is connected to the imaging device 40. When the mechanical arm 20 moves, the mechanical arm 20 can drive the imaging device 40 to move, so that the imaging device 40 can obtain a light signal reflected by an observation object and perform imaging. The observation object may refer to an internal tissue of a human body, or a surgical tool, etc. The light signal reflected by the observation object is transmitted from a surgical incision (that is, a surgical cavity) to the imaging device 40. In the embodiment of the present application, the surgical incision can be assisted by using a surgical tool to establish a channel, for example, establish a channel by using a retractor 30.

One end of the light source providing device is arranged on the support 10, and some or all of the light signals provided by the light source providing device are transmitted to the observation object through the channel established by the retractor 30 (which can be understood as the surgical incision), and are reflected to the imaging device 40 by the observation object, so as to assist the imaging device 40 in imaging.

The controller 50 is electrically connected to the mechanical arm 20, the light source providing device, and the imaging device 40. During the surgery, the controller 50 controls the light source providing device to provide the light source. Here, it should be noted that the time for controlling to provide the light source can be before or after the mechanical arm 20 adjusts the position. This is not limited herein. In some embodiments, the light source providing device may not be controlled by the controller 50, for example, may be manually operated, or may be manually operated or may be controlled by the controller.

The controller 50 first obtains a position and a direction of the surgical incision. For example, the controller 50 can obtain a position and a direction of the retractor 30 (which can be understood as the position and the direction corresponding to the surgical incision). In the following embodiments, the surgical incision, that is, a channel inside and outside the surgical cavity, is explained by taking the channel established by the retractor as an example. In the present application, the position and the direction of the retractor 30 can be identified by using the identification method of the prior art, for example, can be identified by a position sensor, or can be identified by using an image obtained by the imaging device 40. This is not limited herein.

The controller 50 adjusts the position and the direction of the mechanical arm 20 according to the position and the direction of the retractor 30, so that the imaging device 40 can accurately obtain the light signal reflected from the channel established by the retractor 30. Therefore, when the position and the direction of the retractor 30 change, the mechanical arm 20 follows the position and the direction of the retractor 30 to adjust the aerial posture, such as horizontal movement, up and down movement, adjustment of the depression angle and elevation angle, etc.. This facilitates the imaging device 40 to shoot the observation object, and realizes multi-angle imaging, and it is convenient to perform the surgery.

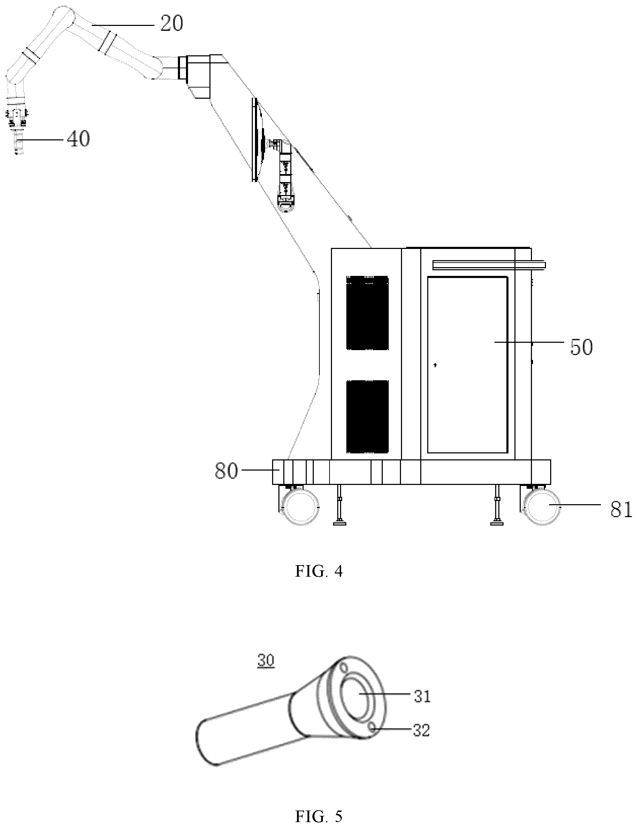

In the present application, for the specific position and direction identification of the retractor 30, reference can be made to the prior art. Details are not described herein. The retractor is described in detail in the corresponding FIGS. 5-7 in the following embodiments.

According to the imaging system 100 for surgical implantation of the embodiment of the present application, the mechanical arm 20 and the imaging device 40 are provided, the direction and the position of the surgical channel can be adjusted and positioned, and the aerial posture of the mechanical arm 20 can be adjusted by using the position and the posture of the retractor 30. This facilitates the shooting of the imaging device 40 connected to the mechanical arm 20, and it is convenient to perform the surgery. In addition, a light source is remotely provided for the imaging device 40 by the light source providing device, so that the imaging device 40 can clearly obtain the image of the observation object at a longer distance. Compared with the existing microscope, in the present application, the light source is provided closer to the surgical position, and the lighting efficiency is greatly improved, so that the image of the observation object can still be clearly obtained when a working distance between the lens of the microscope and the surgical working area is more than 1 meter, and it is convenient to perform the surgery.

In one embodiment of the present application, the imaging system 100 may include a retractor 30.

As shown in FIGS. 5-7, the retractor 30 may be a tubular structure, one end of the retractor 30 is a trumpet-shaped structure that spreads outward from the axis. A first channel 31 is provided in the middle of the retractor 30, and a second channel 32 is provided at the trumpet-shaped end. The second channel 32 is finally connected to the first channel 31, and the human tissue in the surgical cavity is exposed through the channel, so that the light signal reflected by the observation object (the part of the human tissue and the surgical tool, etc.) is obtained by the imaging device 40, and it is convenient to perform imaging.

In the above embodiment, the position and the direction of the retractor mentioned may refer to the orientation of the trumpet-shaped end of the retractor, and the relative spatial position of the trumpet-shaped end, or the distance from the support, etc.

During surgery, the retractor 30 can be placed in the surgical incision to stretch the incision to form a channel connecting the outside world with the surgical cavity inside the human body, such as the first channel 31 and the second channel 32 shown in FIGS. 5-7. The first channel 31 can be used as a channel through which the surgical operator performs the operation, and as a channel for the light reflected by the observation object. The second channel 32 can introduce external light into the first channel 31, and then the external light enters into the surgical incision to illuminate the observation object. The light reflected by the observation object is reflected by the first channel 31 and finally obtained by the imaging device 40.

In some embodiments, a plurality of second channels 32 may be provided. For example, the description in the present application is based on an example of having two second channels 32. In other embodiments of the present application, there may also be a plurality of channels, and it is convenient for the light to enter from multiple directions, and illumination is facilitated. This is not limited herein.

As shown in FIG. 1, in one embodiment of the present application, the mechanical arm 20 has at least six joints 21, and each joint 21 can rotate freely, so that the mechanical arm 20 has at least six degrees of freedom angles of rotation, shooting is performed more freely, there is no limitation on the surgery, and it is conducive to performing the surgery.

According to one embodiment of the present application, the imaging device 40 includes a light filter and an imaging camera. The light filter can obtain the light signal reflected by the observation object and filter out a light signal that meets the preset wavelength in the light signals. For example, the light filter can separate visible light and infrared light, so that the collection of light signals in different bands is implemented, it is convenient to perform imaging according to the band, and it is conducive to implementing optical navigation. It is convenient for long-distance imaging.

In an embodiment of the present application, in order to improve the imaging effect of the imaging camera, the imaging camera is a lens with a high magnification. For example, the lens of the imaging camera can be a combination of an optical zoom and a digital zoom camera to achieve a greater magnification, so that within a range of 1 meter, the imaging camera can clearly obtain the image of the observation object. Compared with the prior art, the distance between the lens and the working area can be increased to prevent the imaging device 40 from blocking another necessary intraoperative imaging device (such as a C-arm machine) and the line of sight of the surgical operator, and it is convenient to perform the surgery.

In one embodiment of the present application, the light source providing device can provide light of variable intensity and can implement remote control. For example, the controller 50 is connected to the light source providing device, and the remote computer is connected to the controller 50 through a network to implement remote control of the light source intensity.

As shown in FIGS. 1-2, the light source providing device may include a light emitting diode 70 (Light Emitting Diode, LED) and a light guide (not shown). The LED is mounted on the support 10, one end of the light guide is arranged opposite to the LED, and the other end is used to connect to the retractor 30. The light signal emitted by the LED is transmitted to the retractor 30, so that the light signal reaches the observation object through the channel.

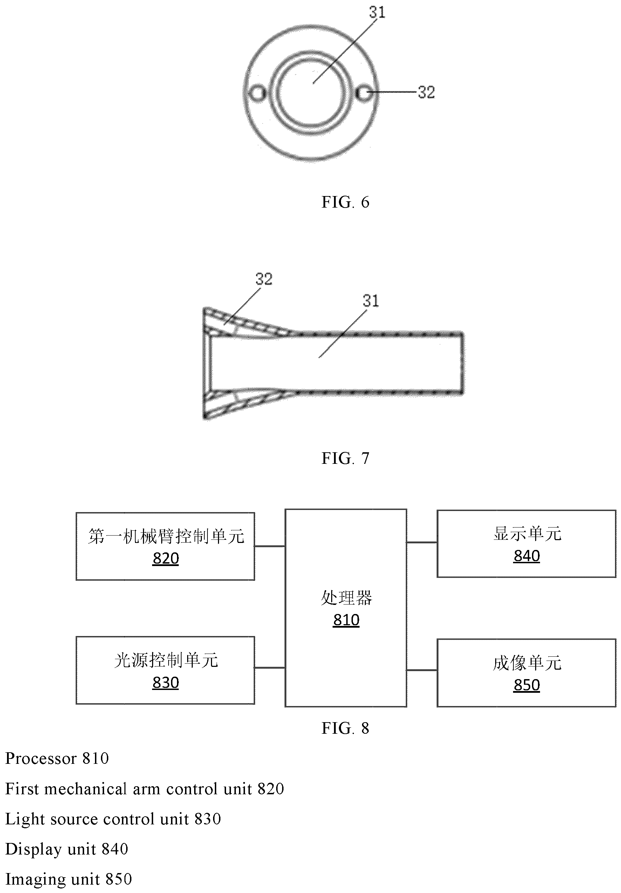

In an embodiment of the present application, as shown in FIG. 7, the end of the light guide can be connected to the second channel 32, and the light enters from the trumpet-shaped end of the retractor 30, exits from the other end, and irradiates the observation object. The inner wall of the retractor 30 can be an inner surface with high reflective properties, and it is convenient to irradiate the observation object with the light introduced from the light guide.

In some embodiments, the light guide can be a fiber optic light guide, and the light emitted by the LED can be collected through the light guide and introduced into the retractor 30. The inner surface of the retractor 30 has a highly reflective inner surface, and the light signal introduced by the light guide is transmitted to the observation object through the highly reflective inner surface. Because the light emitted by the LED is a certain distance from the surgical work area, the light emitted by the LED does not affect the surgery, for example, does not produce shadows. In addition, the use of the light guide facilitates introducing a long-distance light source into the retractor 30, accurately irradiating the observation object with the light source, and facilitates imaging by the imaging device 40.

As shown in FIG. 1, the support 10 may include a fixing frame 11 and a support frame 13. The fixing frame 11 and the support frame 13 may be made of steel. A first end of the support frame 13 is connected to the fixing frame 11 and extends in a direction away from the fixing frame 11. For example, the support frame 13 is at the farthest end of the fixing frame 11 (corresponding to a second end of the support frame). This structure allows the mechanical arm 20 connected to the second end to cover as large a space range as possible, so as to be away from the surgical area during surgery and avoid blocking the line of sight of the surgical operator.

The fixing frame 11 and the support frame 13 in the present application may be two connected objects or may be integrally formed.

When positioning, the approximate position of the fixing frame 11 is first adjusted, and the farthest end of the support frame 13 is turned toward the direction of the operating table (away from the fixing frame 11), so that the farthest end of the support frame 13 is located above the center line of the operating table (and then directly above the patient), and the farthest end is connected to the mechanical arm 20, so that the imaging device 40 on the mechanical arm 20 is located above the patient, and imaging can be easily performed on the observation object.

As shown in FIG. 1, a connecting piece 12 is connected to the farthest end of the support frame 13, and the support frame 13 is connected to the mechanical arm 20 through the connecting piece 12. This structure facilitates the connection of the mechanical arm 20 to the support frame, and it is conducive to the movable connection of the joint 21 of the mechanical arm 20.

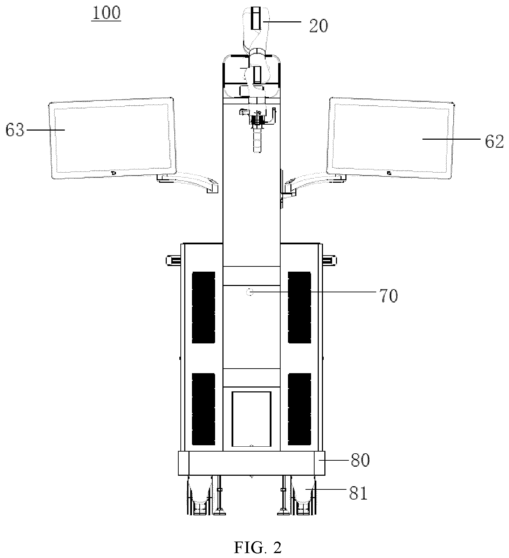

As shown in FIG. 1 and FIG. 2, the imaging system 100 further includes a display, and the display is connected to the controller 50. The controller 50 sends image data of the observation object obtained by the imaging device 40 to the display, and the display further displays the image corresponding to the image data, so that the surgical team can perform analyzing and operation according to the image displayed on the display.

Preferably, there are at least three displays, and at least two of the at least three displays are oriented differently. As shown in FIG. 1, the display includes a first display 61, a second display 62, and a third display 63. The first display 61 is fixed on a host (including the controller 50), and the interface is placed upward, so that the medical staff standing next to the host can observe from this angle. The second display 62 and the third display 63 are respectively arranged on both sides of the support 10, and are symmetrically arranged, and the medical staff on both sides of the support 10 views the interface. This makes it easier for different medical staff to view the same observation object from different angles, and facilitates teamwork to complete the surgery.

In one embodiment of the present application, a base 80 may be provided below the support 10 for fixing the host and the support 10, and a roller 81 may be provided below the base, preferably an automatic roller 81 may be selected, to facilitate the overall movement and positioning of the entire imaging system 100.

With reference to FIG. 8, FIG. 8 is a schematic diagram of system architecture of an imaging system 100 of the embodiment of the present application. As shown in FIG. 8, the architecture diagram includes a processor 810 (corresponding to a controller 50, and the processor may be a part of the controller 50), and a mechanical arm 20 control unit 820 (corresponding to a mechanical arm 20), an imaging unit 850 (corresponding to an imaging device 40 of FIG. 1), a light source control unit 830 (corresponding to a light source providing device), and a display unit 840 (corresponding to a display) that are respectively connected to the processor 810. The mechanical arm 20 control unit 820 may be a combination of the mechanical arm 20 and a plurality of drive units. For example, the drive unit may be a part of the mechanical arm 20. The processor 810 adjusts the free angle of the mechanical arm 20 by controlling the drive unit. The drive unit may be a drive unit such as a motor, a cylinder, or a motor. The imaging unit 850 may include an imaging camera, etc. The light source providing device may include an LED. In addition, the display unit 840 may include a display controller 50 and a display screen. The display controller 50 is connected to the processor. The processor obtains image data of the observation object captured by the imaging unit and sends the image data to the display controller 50. The display controller 50 processes and renders the image, and finally displays the image corresponding to the observation object on the display screen.

The present application further discloses an imaging method for surgical implantation. The method is applied to the imaging system 100 for surgical implantation described in the above embodiment. The specific structure of the imaging system 100 is described in detail in the above embodiment. Details are not repeated herein.

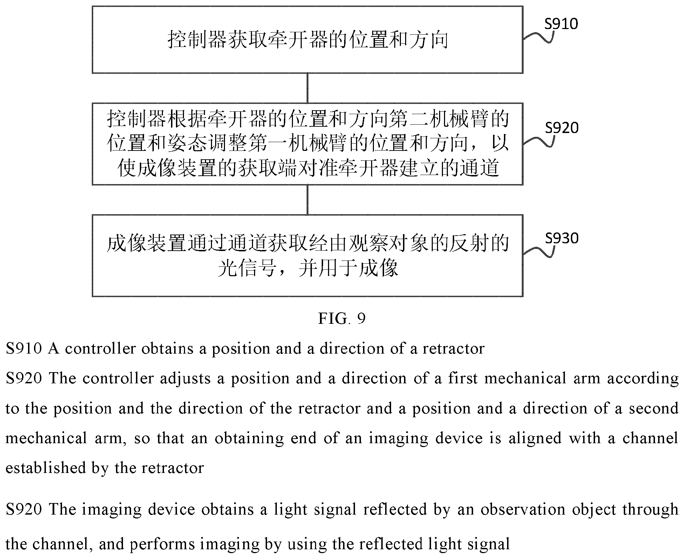

As shown in FIG. 9, the method specifically includes S910-S940.

S910, the controller 50 obtains a position and a direction of the retractor 30. In the embodiment of the present application, the position and the direction of the retractor 30 can be obtained by a distance sensor, such as an infrared sensor, or the position and the direction of the retractor 30 can be obtained by collecting an image by using a depth camera. This is not limited in the present application.

S920, the controller 50 adjusts a position and a direction of the mechanical arm 20 according to the position and the direction of the retractor 30, so that an obtaining end of the imaging device 40 is aligned with a channel established by the retractor 30.

S930, the imaging device 40 obtains a light signal reflected by the observation object through the channel, and performs imaging by using the reflected light signal.

According to the imaging method for surgical implantation of the embodiment of the present application, the direction and the position of the mechanical arm 20 are automatically controlled, so that the imaging device 40 follows the position and the direction of the retractor 30 to perform shooting, and it is convenient to obtain an image; and the mechanical arm can bypass the surgical area, and it is convenient to perform the surgery.

In one embodiment of the present application, the imaging device 40 obtains a light signal of a preset wavelength from the reflected light signal, and performs imaging based on the light signal of the preset wavelength.

According to one embodiment of the present application, the light source provider emits a light signal, and transmits some or all of the light signals to the observation object through the channel, so that the imaging device 40 obtains the light signal reflected by the observation object, improves the clarity of the shooting, and remotely transmits the light signal, to avoid a case in which the light affects the surgical operator.

In addition, the controller 50 obtains imaging data obtained by the imaging device 40, and sends the imaging data to a plurality of displays, so that the display displays the image corresponding to the observation object, and it is convenient for the medical staff to observe the image. The display can be provided at different positions, so that the medical staff at different positions can observe the image of the same observation object on different displays, and it is conducive to performing the surgery.

The functions of each component and the imaging process are described in the above embodiment. For details, refer to the specific description of the imaging system 100. Details are not repeated herein.

According to the imaging method of the embodiment of the present application, the image of the observation object is obtained by controlling the mechanical arm to drive the imaging device 40, and a light source is remotely provided for the imaging device 40 by the light source providing device, so that the imaging device 40 can also clearly obtain the image of the observation object at a longer distance. Compared with the existing microscope, in the present application, the light source is provided relatively closer to the surgical position, and the lighting efficiency is greatly improved, so that the image of the observation object can still be clearly obtained when a working distance between the lens of the microscope and the surgical working area is more than 1 meter, and it is convenient to perform the surgery.

Unless otherwise defined, the technical terms or scientific terms used in the present application should be the common meaning understood by people with general skills in the field to which the present application belongs. The words "first", "second" and similar words used in the present application do not indicate any order, quantity or importance, but are only used to distinguish different components. The words "joint" or "connection" and similar words are not limited to a physical or mechanical connection, but may include an electrical connection, whether direct or indirect. "Up", "down", "left", "right", etc. are only used to indicate relative positional relationships. When the absolute position of the described object changes, the relative positional relationship also changes accordingly.

The above content is the preferred embodiment of the present application. It should be pointed out that for ordinary technicians in this technical field, several improvements and modifications can be made without departing from the principles described in the present application. These improvements and modifications should also be considered within the scope of protection of the present application.

## Claims

1. An imaging system for surgical implantation, comprising:
a support;
a mechanical arm, wherein a first end of the mechanical arm is rotatably connected to the support;
an imaging device, wherein the imaging device is connected to a second end of the mechanical arm, the imaging device rotates freely under the driving of the mechanical arm, and is used to obtain a light signal reflected by an observation object and perform imaging, wherein the light signal reflected by the observation object is transmitted from a surgical incision to the imaging device;
a light source providing device, wherein one end of the light source providing device is arranged on the support, some or all of the light signals provided by the light source providing device are transmitted to the observation object through the surgical incision, and are reflected by the observation object to the imaging device to assist the imaging device in imaging; and
a controller, wherein the controller is electrically connected to the mechanical arm, the light source providing device, and the imaging device, the controller obtains a direction and a position of the surgical incision and makes an adjustment to a posture, and adjusts an aerial posture of the mechanical arm according to a position and a direction of the surgical tool, so that the imaging device can accurately obtain the reflected light signal.

2. The imaging system according to claim 1, further comprising:
a retractor, wherein the retractor is used for placement within the surgical incision to establish a transmission channel inside and outside the surgical incision; and
the controller obtains a position and a direction of the retractor and makes an adjustment to a posture, and adjusts an aerial posture of the mechanical arm according to the position and direction of the retractor, so that the imaging device can accurately obtain the reflected light signal from the channel established by the retractor.

3. The imaging system according to claim 1, wherein the imaging device comprises:
a light filter, wherein the light filter is used to separate the light signal of a preset wavelength; and
an imaging camera, wherein the imaging camera obtains a wavelength within a preset range, and performs imaging based on the wavelength within the preset range.

4. The imaging system according to claim 1 or 2, wherein the mechanical arm has at least six joints to achieve at least six freedom angles of rotation.

5. The imaging system according to claim 1, wherein the support comprises:
a fixing frame; and
a support frame, wherein a first end of the support frame is connected to the fixing frame, a second end extends outward toward the side away from the fixing frame, and the second end of the support frame is connected to the mechanical arm, to increase a spatial range that the mechanical arm can reach.

6. The imaging system according to claim 5, wherein the support further comprises:
a connecting piece, wherein the connecting piece is fixed on the second end of the support frame, and is used to connect the mechanical arm and the support frame.

7. The imaging system according to claim 5 or 6, wherein the light source providing device comprises:
a light emitting diode LED, wherein the LED is installed on the fixing frame, a light-emitting surface of the LED and the second end of the support frame are located on the same side of the fixing frame, and are used to transmit light to the observation object.

8. The imaging system according to claim 7, wherein the light source providing device further comprises:
a light guide, wherein one end of the light guide is arranged opposite to the LED, and the other end is used to transmit the light signal into the surgical incision to the observation obj ect.

9. The imaging system according to claim 8, further comprising a display, wherein there are at least three of the displays, at least two of the at least three displays are oriented differently, and used for displaying an image obtained via the imaging device.

10. The imaging system according to claim 1, further comprising:
a base, wherein the support is fixed on the base, and a plurality of rollers are provided below the base.

11. An imaging method for surgical implantation, wherein the method is applied to an imaging system, the imaging system comprises: a mechanical arm imaging device and a controller, and the method comprises:
obtaining, by the controller, a position and a direction of a retractor, wherein the retractor is used to establish a channel inside and outside a surgical incision to assist the imaging device in imaging;
adjusting, by the controller, a position and a direction of the mechanical arm according to the position and the direction of the retractor, so that an obtaining end of the imaging device is aligned with the channel established by the retractor; and
obtaining, by the imaging device, a light signal reflected by the observation object through the channel, and performing imaging for the observation object by using the reflected light signal.

12. The imaging method according to claim 11, further comprising: obtaining, by the imaging device, an optical signal of a preset wavelength from the reflected optical signal, and performing imaging based on the optical signal of the preset wavelength.

13. The imaging method according to claim 11 or 12, wherein the imaging system further comprises a light source provider, the light source provider emits a light signal and transmits some or all of the optical signals through the channel to the observation object, so that the imaging device obtains the light signal reflected by the observation object.

14. The imaging method according to claim 11, wherein the imaging system further comprises a plurality of displays, and interface orientations of the plurality of displays have at least two or more orientations, and the method further comprises:
obtaining, by the controller, imaging data obtained by the imaging device, and sending the imaging data to the plurality of displays, so that the plurality of displays with different orientations display the image corresponding to the observation object.

## Amended claims

### Amended claims under Art. 19.1 PCT

1. An imaging system for surgical implantation, comprising:
a support;
a mechanical arm, wherein a first end of the mechanical arm is rotatably connected to the support;
an imaging device, wherein the imaging device is connected to a second end of the mechanical arm, and the imaging device rotates freely under the driving of the mechanical arm, and is used to obtain a light signal reflected by an observation object and perform imaging, wherein the light signal reflected by the observation object is transmitted from a surgical incision to the imaging device;
a light source providing device, wherein one end of the light source providing device is arranged on the support, and some or all of the light signals provided by the light source providing device are transmitted to the observation object through the surgical incision, and are reflected by the observation object to the imaging device, to assist the imaging device in imaging;
a retractor, wherein the retractor is used for placement within the surgical incision to establish a transmission channel inside and outside the surgical incision; and
a controller, wherein the controller is electrically connected to the mechanical arm, the light source providing device, and the imaging device, the controller obtains a position and a direction of the retractor and makes an adjustment to a posture, and adjusts an aerial posture of the mechanical arm according to the position and the direction of the retractor, so that the imaging device can accurately obtain the reflected light signal.

2. The imaging system according to claim 1, wherein the imaging device comprises:
a light filter, wherein the light filter is used to separate the light signal of a preset wavelength; and
an imaging camera, wherein the imaging camera obtains a wavelength within the preset range, and performs imaging based on the wavelength within the preset range.

3. The imaging system according to claim 1 or 2, wherein the mechanical arm has at least six joints to achieve at least six freedom angles of rotation.

4. The imaging system according to claim 1, wherein the support comprises:
a fixing frame; and
a support frame, wherein a first end of the support frame is connected to the fixing frame, a second end extends outward toward the side away from the fixing frame, and the second end of the support frame is connected to the mechanical arm, to increase a spatial range that the mechanical arm can reach.

5. The imaging system according to claim 4, wherein the support further comprises:
a connecting piece, wherein the connecting piece is fixed on the second end of the support frame, and is used to connect the mechanical arm and the support frame.

6. The imaging system according to claim 5, wherein the light source providing device comprises:
a light emitting diode LED, wherein the LED is installed on the fixing frame, a light-emitting surface of the LED and the second end of the support frame are located on the same side of the fixing frame, and the LED is used to transmit light to the observation obj ect.

7. The imaging system according to claim 6, wherein the light source providing device further comprises:
a light guide, wherein one end of the light guide is arranged opposite to the LED, and the other end is used to transmit the light signal into the surgical incision to the observation obj ect.

8. The imaging system according to claim 7, further comprising a display, wherein there are at least three displays, and at least two of the at least three displays are oriented differently, and are used for displaying an image obtained via the imaging device.

9. The imaging system according to claim 1, further comprising:
a base, wherein the support is fixed on the base, and a plurality of rollers are provided below the base.
